# EUROPEAN PATENT SPECIFICATION

(11) **EP 4 617 025 B1**
(45) Date of publication and mention of the grant of the patent: **02.09.2026**
(21) Application number: 24162957.5
(22) Date of filing: 12.03.2024
(51) Int. Cl.: B27K 5/00, F23J 15/00, A61L 9/22, B01D 45/00, B03C 3/16

(54) **A METHOD AND A SYSTEM FOR PURIFYING AN OFF-GAS STREAM COMPRISING PARTICLE MATTER AND VOLATILE ORGANIC COMPOUNDS**
VERFAHREN UND SYSTEM ZUR REINIGUNG EINES ABGASSTROMS MIT PARTIKELMATERIAL UND FLÜCHTIGEN ORGANISCHEN VERBINDUNGEN
PROCÉDÉ ET SYSTÈME DE PURIFICATION D'UN FLUX DE GAZ DE DÉGAGEMENT COMPRENANT UNE MATIÈRE PARTICULAIRE ET DES COMPOSÉS ORGANIQUES VOLATILS

(43) Date of publication of application: 17.09.2025
(73) Proprietor: Valmet Technologies Oy, 02150 Espoo (FI)
(72) Inventor: Katainen, Janne, 33900 Tampere (FI); Rintanen, Jaakko, 33900 Tampere (FI); Silvennoinen, Jaani, 33900 Tampere (FI)
(74) Representative: Berggren Oy

(56) References cited:
- CN-A- 107 998 859
- CN-A- 108 579 300
- CN-B- 105 561 776
- US-A1- 2005 229 780
- US-A1- 2022 023 880

## Description

### Technical field

This specification relates to a method for purifying an off-gas stream comprising particle matter and volatile organic compounds as well as to a system for such. Further, the specification relates to use of the system.

### Background

Wet electrostatic precipitation (WESP) is widely used in mechanical wood industry to remove dust, volatile organic compounds (VOCs) and formaldehyde from the off-gases originating for example from wood chip dryer and panel press. Achievable VOC reduction with WESP technology is typically only up to 10 %. If higher reduction is needed, thermal oxidizers, such as regenerative thermal oxidizers (RTO), are typically used. However, RTO plants are expensive and utilize external fuel such as oil or gas.

WESP is also used in mineral wool industry to remove dust, phenol, formaldehyde and volatile binder components from the off-gases originating for example from the forming area and the curing oven. Achievable VOC reduction with WESP technology is poor and difficult to predict.

Therefore, alternative methods for VOC removal are needed.

Prior art document US2005/229780 A1 discloses a pollution control system associated with a wood product dryer operation. Prior art documents CN105561776B, CN107998859A and CN108579300A disclose systems for purification of exhaust gases comprising VOCs.

### Summary

This specification aims to provide an improved method and a system for VOC removal from off-gases. With the solution disclosed herein the need to use RTO downstream of the WESP system may be avoided, as the emission limits of the VOCs and formaldehyde contained by the off-gases are reachable by WESP alone.

According to an embodiment, a method for purifying an off-gas stream comprising particle matter and volatile organic compounds according to claim 1 is provided.

According to another embodiment, a system for purifying an off-gas stream comprising particle matter and volatile organic compounds according to claim 6 is provided.

Further, use of the system as disclosed above for purifying an off-gas stream comprising particle matter and volatile organic compounds is provided.

### Brief description of the drawings

- Fig. 1: illustrates, by way of an example, a system configured to purify a gas stream comprising particle matter and volatile organic compounds by implementing a method disclosed herein and
- Fig. 2: illustrates, by way of an example, an overview of a WESP,
- Fig. 3: illustrates, by way of an example, a system with a WESP comprising two quenches and configured to purify a gas stream comprising particle matter and volatile organic compounds by implementing a method disclosed herein.

The figures are schematic. The figures are not on any particular scale.

### Detailed description

The solution is described in the following in more detail with reference to some embodiments, which shall not be regarded as limiting.

Unit of temperature expressed as degrees C corresponds to °C. The following reference numbers and denotations are used in this application:

| | |
|---|---|
| 100, 300 | system |
| 101, 201 | off-gas stream |
| 102 | cyclone |
| 103 | fan |
| 104, 204, 304 | WESP |
| 105, 205, 305 | treated off-gas stream |
| 106, 206 | quench of the WESP |
| 107, 307 | chemical dosing system |
| 204a | inlet zone |
| 204b | precipitation zone |
| 204c | outlet zone |
| 301a | first off-gas stream |
| 301b | second off-gas stream |
| 302a | first cyclone |
| 302b | second cyclone |
| 303a | first fan |
| 303b | second fan |
| 306a | first quench |
| 306b | second quench |

Mechanical wood industry or mechanical forest industry (or wood products industry) includes sawmill, plywood, chipboard, fibreboard, wood construction products and construction industries.

Chipboard, also called particleboard, comprises dried wood chips which are glued together with a resin which cures under the influence of high pressure and heat. The wood chips are dried in heated rotating dryers. Resins and other additives are mixed with the dried wood chips and a mat of resonated chips is formed and cured in a hot press applying high pressure.

Oriented strand board (OSB) comprises dried wood strands which are glued together with a resin which cures under the influence of high pressure and heat. The manufacturing process is similar to that of chipboard.

Fibreboard includes the terms medium density fibreboard (MDF), low density fibreboard (LDF), high density fibreboard (HDF) and softboard. Fibreboard is produced from dry wood fibres that are glued together with a resin which cures under the influence of high pressure and heat. Wood fibres are mainly derived from roundwood, which is chipped or flaked and refined in a thermomechanical pulping process. The refined wet fibres are dosed with resin and additives and dried in tube dryers. The forming of the mat and the pressing process are similar to that of the chipboard and oriented strand board.

Within context of this disclosure, term "off-gas" refers to a gas that is produced as a by-product of an industrial process or that is given off by a manufactured object or material. The off-gases originating from mechanical wood industry differ from gas streams obtained by combustion (flue gases) in terms of the amount of the VOCs. The amount of the VOCs in combustion gases is remarkably lower or below detection limits when compared to the off-gases originating from mechanical wood industry. Terms "off-gas stream" and "gas stream" are used interchangeably throughout the disclosure.

Off-gases originating from the dryers and presses used for wood-based board or panel production contain inorganic and organic particle matter (dust), VOCs and formaldehyde, that are pollutants, i.e., substances that have undesired environmental effects and/or are harmful or even dangerous to human health. Also, off-gases originating from mineral wool industry contain VOCs. Such off-gases can contain up to 500 mg/Nm³ VOCs. Therefore, the off-gases need to be purified prior to their release into atmosphere.

Depending on the origin the off-gases may further comprise nitrogen oxides (NOx), such as nitrogen monoxide (NO). For example, off-gas originating from a dryer in wood industry may contain NOx, as flue gas may be used in drying. Off-gas originating from a press typically does not contain NOx.

The electrostatic precipitation (ESP) technique is widely used in the gas purification industry for filtering out impurities (particles) in gases. The wet electrostatic precipitation (WESP) is particularly advantageous, since it is efficient at charging and collecting sub-micrometer particles. An ESP removes aerosols from a gas stream due to forces induced by strong electric fields. The addition of spray of water mist into the ESP in a WESP system enhances the particle growth in diameter and the particle charging, further the surface area of the aqueous filmic layer on the collecting surfaces is increased. Currently, WESP is efficient for removal of particulate impurities, but the technique needs further improvements for filtration of gaseous impurities, such as VOCs.

With current WESP processes, VOC and aldehyde reduction (i.e., decrease in the amounts thereof) from the gas streams is based on scrubber washing, condensation and post oxidation. Post oxidation in WESP is caused by high voltage (up to 130 kV peak) inducing characteristic corona effect to take place. The ozone thus formed is capable of oxidizing VOCs and formaldehyde. However, the reduction in the amounts of the VOCs in the gas stream obtainable with the current WESP processes typically is only up to 10 %. When further decrease is needed, thermal oxidizer such as regenerative thermal oxidizer (RTO) is used downstream of the WESP system. However, RTO systems are expensive to install and they also have high running costs due to both the additional fuel and the required maintenance. Moreover, because of the additional fuel used by the RTO, it does not represent very sustainable solution.

Current emission limits placed by certain nations, such as China and the USA, require use of RTO systems. With present WESP-technology VOC emissions of 100-150 mg/Nm³ are typically achievable. In the US and China the VOC limits presently are 50-70 mg/Nm³ and 60-100 mg/Nm³, respectively. Regarding formaldehyde, emissions of 10 mg/Nm³ are achievable with the current technology. In China formaldehyde emission of below 5 mg/Nm³ is at present required. Thus, in order to avoid the usage of RTO systems, WESP removal of VOCs and formaldehyde needs to be improved. The above disclosed values apply to emissions from dryers or dryer and press combinations in mechanical wood industry. Typically the emissions from a dryer may be twice of that of a press.

Off-gases originating from a dryer and/or a press in mechanical wood industry typically contain α-pinene and β-pinene. Major part of the VOCs contained by the off-gas may be composed of α-pinene and β-pinene. Due to high vapor pressure of α-pinene and β-pinene, their absorption (condensation) in water is obtainable only at low temperatures, such as at 0 degrees C or even lower. Thus, a chemical reaction and/or very low temperature is needed for removing α-pinene and β-pinene from the off-gases.

Typical gas line (or gas conduit) configuration leading from the origin of the off-gas to the WESP comprises a fan, and very often also a cyclone. The cyclone may be arranged prior to the fan in the direction of the gas flow, or vice versa. Purpose of the fan is to provide suction such that the gas remains in the gas line. Thus, the fan compensates for the pressure drop caused by the components of the gas line. Cyclone, also called cyclone separator, is as a mechanical purifier separating solid or liquid particles from a gas stream. Cyclone operates based on the principle of centrifugal force, which causes the particles to move towards the outer wall of the cyclone, while the clean gas flows through the center.

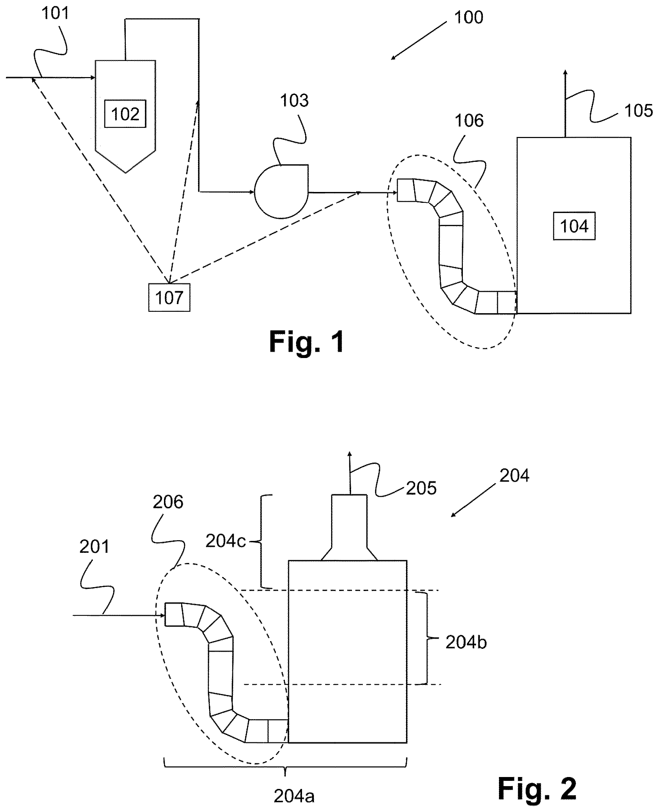

In the direction of gas flow, WESP is arranged after the fan and also after the cyclone, when cyclone is present. In some cases, a pre-scrubber may be arranged after the fan and the optional cyclone, prior to the WESP. An exemplary illustration of a WESP is presented in Figure 2. Typical WESP 204 comprises an inlet zone 204a. The inlet zone 204a is equipped with an inlet for the off-gas 201 to be purified. The WESP also comprises a precipitation zone 204b. Within the precipitation zone 204b the off-gas 201 is configured to be purified following the principles of electrostatic precipitation. Therefore, the WESP 204 comprises discharge electrodes and collecting surfaces. The collecting surfaces are also electrodes. Thus, the discharge electrodes and collecting surfaces are referred to simply as electrodes, when considered feasible. The electrodes are for electrically precipitating the off-gas 201. The electrodes are arranged within the precipitation zone 204b of the WESP. The WESP 204 also comprises an outlet zone 204c for letting out purified off-gas 205. In use, the off-gas 201 that is to be purified in WESP flows from the inlet zone 204a through the precipitation zone 204b to the outlet zone 204c. Therefore, the precipitation zone 204b is arranged in between the inlet zone 204a and the outlet zone 204c of the WESP.

The inlet zone 204a of the WESP comprises a quench 206, i.e. a quench of the WESP. The purpose of the quench is to pre-moisten and cool down the off-gas to be purified to a saturation point or dew point temperature. The quench comprises integrated nozzles for providing liquid, i.e., quench water for moistening and cooling the gas to be purified. The quench may have a length of from 5 to 20 m. The quench water provided to the gas stream is arranged to flow from the quench to the bottom of the WESP and finally into a tank located below the wet absorber located downstream of the quench. Thus, the quench water is contained in the water circulation of the WESP.

In the quench at least most of the off-gas is saturated to the dew point or even below it. This means that the temperature of the gas is lowered for example from 130 degrees C to 70 degrees C, or from 75 degrees C to 50 degrees C. The dew point is to be reached in a short residence time by using excess quench water. During quenching also particles, mainly coarse size particles, may be collected from the off-gas. Temperature of the gas may be lowered even more, especially within context of this disclosure. This may be beneficial taking into account the above discussed high vapor pressure of α-pinene and β-pinene.

The inlet zone of the WESP also comprises a wet absorber. The wet absorber is located downstream to the quench. The wet absorber also functions as a gas distributor and comprises gas distributor screen(s) for distributing the gas stream to be purified. The off-gas flows from the quench through the wet absorber, wherein at least some of the water-soluble contaminants are washed. Condensed tars, essential oils and some of the tarry substances comprised by the off-gas may be precipitated at the wet absorber. The wet absorber comprises nozzles for spraying absorbent water into the off-gas. Preferably the nozzles are high efficiency spraying nozzles that are used for spraying the water homogeneously over the whole section at low off-gas velocity (1-3 m/s). Efficiency of the wet absorber is based on optimal distribution of water droplets, size of the droplets and the residence time. At least some of the dust and particles comprised by the off-gas to be purified may also be precipitated in the wet absorber.

The precipitation zone of the WESP comprises the electrostatic precipitator, which in the case of WESP naturally is a wet one. The wet electrostatic precipitator comprises an electrostatic field wherein remaining solids, aerosols and condensed hydrocarbons of the off-gas are precipitated. In an example, the wet electrostatic precipitation system comprises discharge electrodes with a high actual corona formation and precipitation surfaces designed as grounded collecting electrodes. The discharge electrodes may be wire electrodes. The grounded collecting electrodes may be hexagonal honeycombs or may have annular shape. In the wet electrostatic precipitator the precipitated substances flow along the precipitation surfaces over the wet absorber to the filter bottom and into a tank located below the wet absorber.

In the electrostatic precipitator a discharge electrode emits electrons which collide with the gas molecules and charge them negatively. These ionized molecules then aim for charge equalization on a positive collecting electrode, wherein they collide together with the particles and foist their negative charge on the particles. On the arrival of the ionized molecules and particles at the positive collecting electrode a charge flow-off takes place. The electrostatic precipitator is used for precipitating particles (dust, aerosols, blue haze) and for oxidation of formaldehyde, phenols and hydrocarbons.

As already mentioned, after the electrostatic precipitation stage, certain post-oxidation caused by high-voltage (up to 130 kV peak) characteristic corona effects occurs, thus causing certain oxidation of VOCs, formaldehyde and/or phenols with ozone.

After the purified off-gas leaves the precipitation zone it reaches the outlet zone, i.e., a stack. At the inlet of the stack the purified off-gas may pass a droplet separator with twist air effect, mainly to remove droplets during the field flushing time. The droplet separation may be approximately 95 %. However, use of the droplet separator is not necessary. The purified off-gas is discharged via the stack.

This specification aims to provide an improved method and a system for removal of VOCs from off-gases. With the solution disclosed herein the need to use RTO downstream of the WESP system may be avoided, as the emission limits of the VOCs and formaldehyde contained by the off-gases are reachable by WESP alone. Particularly, the method and the system are for removal of VOCs from off-gases originating from mechanical wood industry or mineral wool industry. The off-gas stream originating from mechanical wood industry may particularly originate from a dryer and/or a press in mechanical wood industry. The dryer and/or the press in mechanical wood industry may be used for wood-based board or panel production. The dryer may be a wood chip dryer and the press may be a panel press. The off-gas stream originating from mineral wool industry may particularly originate from a forming area and/or a curing oven of a mineral wool plant.

A method for purifying an off-gas stream is provided. The off-gas stream comprises particle matter and volatile organic compounds. The off-gas stream comprises off-gas originating from a dryer and/or a press in mechanical wood industry or off-gas originating from a forming area and/or a curing oven of a mineral wool plant.

The method comprises supplying ozone as an oxidizing chemical into the off-gas stream in order to oxidize volatile organic compounds comprised by the off-gas stream, and subjecting the off-gas stream to wet electrostatic precipitation. The ozone is supplied in a gas phase, i.e., in gaseous form. Thus, both the off-gas stream and the ozone as the oxidizing chemical are in gas phase. Supplying the ozone in gas phase enables good enough mixing of the gases and provides sufficient reaction time between the VOC and the ozone before wet conditions.

Ozone (O₃) as the oxidizing chemical is capable of oxidizing volatile organic compounds comprised by the gas stream into a less harmful form and/or into a form that is removable from the gas stream by the WESP system. The ozone is capable of oxidizing the volatile organic compounds into carbon dioxide (CO₂) and water. The ozone is capable of breaking the volatile organic compounds into compounds of increased water solubility, such as organic acids, which have a low vapor pressure in aqueous solution.

Ozone as non-chlorinated oxidizing chemical is preferred over the chlorinated ones, as it does not cause accumulation of chlorine-containing substances to the washing liquid.

Ozone is supplied to the off-gas stream prior to subjecting the off-gas stream to wet electrostatic precipitation, i.e., prior to feeding the off-gas stream to a wet electrostatic precipitator. Preferably, the ozone is supplied to the off-gas stream prior to any water injection or humidification of the off-gas stream. The ozone may be supplied to the off-gas stream at one or more locations. The ozone may be supplied to the off-gas stream prior to feeding the off-gas stream to a quench of a wet electrostatic precipitator or a pre-scrubber preceding the wet electrostatic precipitator, when a pre-scrubber is present. Alternatively or additionally, the ozone may be supplied to the off-gas stream prior to feeding the off-gas stream to a fan preceding the wet electrostatic precipitator. Alternatively or additionally, the ozone may be supplied to the off-gas stream prior to feeding the off-gas stream to a cyclone preceding the wet electrostatic precipitator.

The ozone is supplied by a chemical dosing system. The chemical dosing system may comprise nozzles for supplying the ozone into the off-gas stream. The chemical dosing system is connected to an ozone generator. The chemical dosing system may comprise a container (a reservoir) for containing ozone formed by the ozone generator. The ozone from the container may be supplied into the off-gas stream even without the need to start up the ozone generator. Thus, the ozone contained by the container may serve as a buffer for a short-term higher need of ozone.

Ensuring long enough reaction time for the oxidative reaction is of utmost importance for the method and the system disclosed herein. The residence time for the oxidation of the volatile organic compounds may be at least 1 second. The residence time is preferably from 1 to 3 seconds, or more preferably from 2 to 3 seconds. The residence time may also be referred to as a retention time. The residence time refers to the time from injection point of the ozone to the quench of the WESP or to the pre-scrubber preceding the WESP, if such a pre-scrubber is present.

Long enough residence time helps in minimizing the consumption of ozone. Other parameters helping in minimizing the consumption of ozone as the oxidizing chemical include low enough reaction temperature and lowest possible amount of NOx comprised by the off-gas. NOx present in the off-gas consumes ozone. Therefore, the higher the content of NOx in the off-gas, the higher the need for ozone to gain comparable VOC oxidation.

O₃/NO molar ratio of higher than 0.5 is preferred for effective VOC oxidation. With O₃/NO molar ratio of higher than 0.5, the amount of ozone is high enough for oxidizing VOCs in addition to NOx oxidation. Further, the reaction temperature should be at most 130 degrees C. The reaction temperature may be from 0 to 130 degrees C, such as from 20 to 130 degrees C. In optimal conditions the off-gas contains substantially no NOx. In such a case VOC reduction of over 90 % is obtainable.

The method may further comprise determining the content of the volatile organic compounds in the off-gas stream on-line and adjusting the amount of the supplied ozone on the basis of the content of the volatile organic compounds. For example, the content of the volatile organic compounds may be measured in the outlet zone of the WESP, i.e., the stack. Alternatively or additionally the method may comprise indirectly determining the content of the volatile organic compounds for example by measuring parameter(s) from the quench water and/or the absorbent water, and adjusting the amount of the ozone on the basis of the content of the volatile organic compounds in the gas stream.

Outcome of the disclosed method is a treated, i.e., purified gas stream, wherein the amounts of particle matter and volatile organic compounds are decreased when compared to the situation prior to implementing the method. Particularly, reduction in the amounts of the volatile organic compounds in the gas stream may be at least 10 %, for example 10-70 % or 30-70 %. However, lower or higher reductions may also be desired. In any case, achieving the emission limits with as low consumption of chemicals and electrical power as possible is the situation that is aimed at.

Figure 1 illustrates an exemplary system 100 for implementing the method as disclosed herein. The system 100 is for purifying an off-gas stream 101. The off-gas stream 101 comprises particle matter and volatile organic compounds.

The off-gas stream 101 comprises off-gas originating from a dryer and/or a press used for wood-based board or panel production or off-gas originating from a forming area and/or a curing oven of a mineral wool plant.

The system 100 comprises a wet electrostatic precipitator 104. The WESP comprises an inlet zone, a precipitation zone and an outlet zone. The exemplary details of the WESP are presented in Fig. 2 and discussed above. The wet electrostatic precipitator is suitable for removal of particle matter and volatile organic compounds from an off-gas stream. The system further comprises a chemical dosing system 107 arranged upstream of the wet electrostatic precipitator for supplying ozone in a gas phase to the off-gas stream. The system further comprises a production unit for producing the gas stream comprising particle matter and volatile organic compounds. The production unit comprises a dryer and/or a press in mechanical wood industry, or a forming area and/or a curing oven of a mineral wool plant.

According to an embodiment, the system further comprises a fan 103 and an optional cyclone 102 arranged upstream of the WESP 104, particularly upstream of the quench 106 of the WESP. A gas line (or gas conduit) leads from the origin of the off-gas, i.e., the production unit, to the WESP, the gas line comprising the fan 103 and optionally the cyclone 102. Figure 1 illustrates a system comprising both the cyclone 102 and the fan 103. When both the cyclone 102 and the fan 103 are present, the cyclone 102 may be located upstream of the fan 103, as illustrated in Fig. 1, or vice versa.

The chemical dosing system 107 may be arranged upstream of a quench 106 or a pre-scrubber of the WESP, if the pre-scrubber is present. Alternatively or additionally, the chemical dosing system 107 may be arranged upstream of a fan 103 preceding the WESP. Alternatively or additionally, the chemical dosing system 107 may be arranged upstream of a cyclone 102 preceding the WESP.

The chemical dosing system 107 is arranged to supply ozone in gaseous form. The chemical dosing system 107 may be arranged to supply ozone as the oxidizing chemical to the gas stream at one or more locations. For example, a single chemical dosing system may be arranged to supply ozone into all locations where the chemical is needed. Alternatively, the system may comprise several chemical dosing systems.

According to a preferable embodiment the chemical dosing system 107 is arranged such that residence time for the oxidation of the volatile organic compounds is arranged to be at least 1 second. The chemical dosing system 107 may be arranged such that residence time for the oxidation of the volatile organic compounds is arranged to be from 1 to 3 seconds.

As discussed above, the inlet zone of the WESP comprises an inlet for the off-gas stream to be purified. The inlet zone comprises at least one quench 106. The quench 106 is arranged to pre-moisten and cool down the off-gas stream to be purified to a saturation point or dew point temperature. The quench comprises integrated nozzles for providing liquid, i.e., quench water, for moistening and cooling the gas stream to be purified. As already discussed, the temperature of the gas stream may be lowered for example to 50 degrees C or even lower.

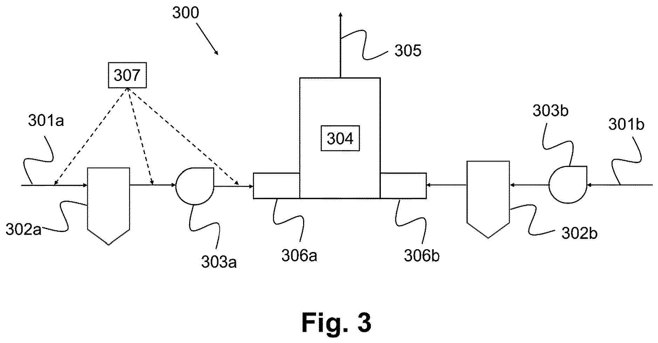

The inlet zone may comprise several quenches. For example, the inlet zone may comprise two quenches, as illustrated in Figure 3. Each of the several quenches may be arranged to serve as inlets for off-gas streams from different origins. Alternatively, an off-gas stream from a single origin may be divided between several quenches.

Fig. 3 illustrates another exemplary system 300 for implementing the method as disclosed herein. The system 300 is for purifying first off-gas stream 301a and second off-gas stream 301b. The first off-gas stream 301a and the second off-gas stream 301b comprise particle matter and volatile organic compounds.

As mentioned above, the first off-gas stream 301a and the second off-gas stream 301b may originate from different origins or they may originate from a single origin.

The system 300 comprises a wet electrostatic precipitator 304. The WESP comprises an inlet zone, a precipitation zone and an outlet zone. The inlet zone of the WESP comprises first quench 306a and second quench 306b. The system further comprises a chemical dosing system 307 arranged upstream of the wet electrostatic precipitator for supplying ozone in a gas phase to the off-gas stream. The system further comprises a production unit or production units for producing off-gas stream comprising particle matter and volatile organic compounds. The production unit comprises a dryer and/or a press in mechanical wood industry, or a forming area and/or a curing oven of a mineral wool plant.

In the exemplary illustration of Fig. 3, the system 300 further comprises first cyclone 302a, second cyclone 302b, first fan 303a and second fan 303b arranged upstream of the WESP 304, particularly upstream of the first quench 306a and the second quench 306b of the WESP, respectively. First gas line (or gas conduit) leads from the origin of the off-gas, i.e., the production unit, to the WESP, the first gas line comprising the first fan 303a and the first cyclone 302a. Second gas line (or gas conduit) leads from the origin of the off-gas, i.e., the production unit, to the WESP, the second gas line comprising the second cyclone 302b and the second fan 303b. In the system 300 illustrated in Fig. 3 the second fan 303b is located upstream of the second cyclone 302b.

In the system 300 illustrated in Fig. 3, the chemical dosing system 307 is arranged to provide ozone to the first gas line leading to the first quench 306a. However, the same chemical dosing system 307 (or another chemical dosing system) may also be arranged to provide ozone to the second gas line leading to the second quench 306b.

Although the system illustrated in Fig. 3 shows two quenches, the system may also be implemented such that the system comprises two or more gas lines (or gas conduits) but only one quench. Thus, the gas streams from all of the gas lines is fed into one common quench of a WESP. The chemical dosing system may be arranged to provide ozone to one or more of the gas lines, according to the prevailing needs.

In an example, the WESP may comprise a first quench for an off-gas stream originating from a dryer and a second quench for an off-gas stream originating from a press. In another example, the WESP may comprise a first quench for an off-gas stream originating from first dryer/press and a second quench for an off-gas stream originating from second dryer/press. Alternatively, as described above, such examples may only contain one common quench instead of separate quenches. As the VOC contents and/or the NOx contents of the off-gases from the dryer and the press, or the first dryer/press and the second dryer/press may vary, providing separate gas lines to the quench enables optimizing the consumption of ozone. The amount of the ozone supplied by the chemical dosing system to the gas lines may be adjusted separately, based on the VOC contents and/or the NOx contents of the gases to be treated.

Alternatively, a single gas line originating from an origin of the off-gas may be divided into two or more gas conduits. Each of the gas conduits may have their own fan and/or cyclone. In case of two conduits, the off-gas stream contained by only one of the conduits may be supplied with ozone. By treating only partial off-gas stream optimal conditions for VOC oxidation may be provided. The partial off-gas stream may be dimensioned according to the prevailing needs. By oxidizing only a partial off-gas stream it is possible to oxidize only the amount of the off-gas that is necessary for reaching the prevailing emission limits.

Within the precipitation zone of the WESP the off-gas is configured to be purified following the principles of electrostatic precipitation. The precipitation zone comprises discharge electrodes and collecting surfaces, which also act as electrodes.

The outlet zone is configured for letting out a treated gas stream. The gas stream fed to the system is arranged to be treated in the WESP in order to obtain a treated gas stream. In the WESP, the inlet zone, the precipitation zone and the outlet zone are arranged such that the off-gas stream flows from the inlet zone through the precipitation zone to the outlet zone. Therefore, the precipitation zone is arranged in between the inlet zone and the outlet zone of the WESP.

The system may further comprise at least one sensor configured to determine on-line the content of the volatile organic compounds in the off-gas stream. The at least one sensor may be arranged as part of the outlet zone, i.e., stack of the WESP. Further, the system may comprise a processor configured to control the chemical dosing system by taking into account the content of the volatile organic compounds in the off-gas stream determined by the at least one sensor.

The method and the system disclosed herein provides improvement when compared to conventional scrubbing process for the VOC and particle matter removal. The conventional scrubbing technology is only capable of removing particles having a diameter of a micrometer scale from the gas stream, whereas with wet electrostatic precipitation particles having a sub-micrometer diameter (even below 0.1 µm) can be removed. The efficient removal of particle matter in scrubbers may only be achievable in multi-stage scrubbers, thus requiring high consumption of energy and water. Further, regarding the VOC removal, the VOCs with lower solubility require larger scrubber, thus meaning higher investment and operational costs. However, even the multi-stage scrubbers are not comparable with WESP technology in terms of separation efficiency.

Investment costs for chemical dosing system according to this disclosure will be much less when compared to an RTO plant combined with the traditional WESP technology. Operation costs for the WESP with the chemical dosing system are expected to be about the same level or less than those for the system with RTO.

Further, oxidation of the VOCs by ozone provided by the chemical dosing system as disclosed herein when compared to a system with RTO is more flexible. When the RTO must be practically all the time in standby mode, the ozone generator can be switched off for a period of time (such as for hours or days) when there is no need for ozone as the oxidizing chemical. When the need for ozone arises, the ozone generator can be switched on in seconds. Energy can be saved by switching the ozone generator off. With the RTO some energy is consumed even in the standby mode.

Further, if gas streams with unexpectedly high VOC contents are to be purified, the chemical dosing system comprising an ozone reservoir as described earlier provides flexibility and may be used to supplement the ozone provided by the ozone generator. Or alternatively, when the need for ozone is low and lasts for a short period of time, the ozone reservoir of the chemical dosing system may be utilized without needing to switch on the ozone generator. Such flexibility and adjustability is not obtainable by the systems utilizing RTO, at least not in such a prompt manner.

With the method and the system disclosed herein the VOC emission limits are reachable with minimal consumption of ozone as the oxidizing chemical. This also means that minimal amount of liquid oxygen is needed for its production as well as minimum amount of electrical power is consumed by the ozone generator. Further, use of a smaller ozone generator for the oxidizing reaction to take place may be enabled, thereby reducing investment costs.

### Examples

An exemplary calculation for a fibreboard plant producing an off-gas flow of 100 Nm³/s, the off-gas flow containing 100 mg/Nm³ of VOC is provided in Table 1. The VOC emission limit is 70 mg/Nm³. Temperature of the gas is 65 °C. Ozone as the oxidizing chemical is fed into the entire off-gas flow prior to quench and the entire ozone treated off-gas flow is subsequently directed into a WESP.

**Table 1.**

| **Substance** | **Amount** | **Unit** |
|---|---|---|
| Off-gas flow | 100 | Nm³/s dry |
| VOC | 100 | mg/Nm³ |
| VOC in | 10 | g/s |
| VOC in | 0,073 | mol/s |
| NOx in as NO₂ | 250 | mg/Nm³ |
| NO from incoming NOx | 100 | % |
| NOx in as NO | 163,0 | mg/Nm³ |
| NO in | 16,3 | g/s |
| NO in | 0,543 | mol/s |
| Temperature | 65 | °C |
| | | |
| O₃/VOC molar ratio | 4,8 | |
| VOC reduction | 30 | % |
| | | |
| VOC out | 70 | mg/Nm³ |
| | | |
| O₃ needed | 42 | kg/h |
| Electricity | 417 | kW |

As illustrated in Table 1, 30% VOC reduction for the off-gas flow containing 16,3 g/s NOx requires 42 kg/h ozone (O₃/VOC molar ratio 4,8) and 417 kW electrical power.

Another exemplary calculation is presented in Table 2. The WESP is fed with two gas streams. The first gas stream originates from first dryer and the second gas stream originates from second dryer of a fibreboard plant. The first gas stream and the second gas stream differ in their NOx content, because the gases used in the dryers for drying have different origins and thus different NOx contents. In this example ozone as the oxidizing chemical is fed into the off-gas stream with lower NOx content. The off-gas has a total flow of 100 Nm³/s and the total incoming VOC content of the off-gas is 100 mg/Nm³. Again, the VOC emission limit is 70 mg/Nm³ and temperature of the gas is 65 °C.

**Table 2.**

| **Substance** | **Total** | **O₃ injection (50%)** | **No O₃ injection (50%)** | **Unit** |
|---|---|---|---|---|
| Off-gas flow | 100 | 50 | 50 | Nm³/s dry |
| VOC | 100 | 100 | 100 | mg/Nm³ |
| VOC in | 10 | 5 | 5 | g/s |
| VOC in | 0,073 | 0,037 | 0,037 | mol/s |
| NOx in as NO₂ | 250 | 134 | 366 | mg/Nm³ |
| NO from incoming NOx | 100 | 100 | 100 | % |
| NOx in as NO | 163 | 87,4 | 238,7 | mg/Nm³ |
| NO in | 16,3 | 4,4 | 11,9 | g/s |
| NO in | 0,543 | 0,146 | 0,398 | mol/s |
| Temperature | 65 | | | °C |
| | | | | |
| O₃/VOC molar ratio | 2,4 | 4,8 | | |
| VOC reduction | 30 | 60 | | % |
| | | | | |
| VOC out | 70 | 40 | 100 | mg/Nm³ |
| | | | | |
| O₃ needed | 20 | | | kg/h |
| Electricity | 200 | | | kW |

When compared to the situation illustrated in Table 1, reduction in the consumption of electrical power (200 kW vs. 417 kW) is achieved. Further, also consumption of ozone is less than half of that in the example of Table 1.

## Claims

1. A method for purifying an off-gas stream (101, 201, 301a, 301b) comprising particle matter and volatile organic compounds, wherein the method comprises
- supplying ozone into the off-gas stream (101, 201, 301a, 301b) in order to oxidize volatile organic compounds comprised by the off-gas stream, and
- subjecting the off-gas stream (101, 201, 301a, 301b) to wet electrostatic precipitation,
wherein the ozone is supplied in a gas phase,
the ozone is supplied into the off-gas stream (101, 201, 301a, 301b) prior to feeding the off-gas stream (101, 201, 301a, 301b) to a wet electrostatic precipitator (106, 206, 306a, 306b), and
the off-gas stream comprises off-gas originating from a dryer and/or a press used for wood-based board or panel production or off-gas originating from a forming area and/or a curing oven of a mineral wool plant.

2. The method according to claim 1, wherein the ozone is supplied to the off-gas stream (101, 201, 301a, 301b) prior to any water injection or humidification of the off-gas stream.

3. The method according to claim 1 or 2, wherein the ozone is supplied to the off-gas stream (101, 201, 301a, 301b)
- prior to feeding the off-gas stream (101, 201, 301a, 301b) to a quench of the wet electrostatic precipitator (106, 206, 306a, 306b) or a pre-scrubber preceding the wet electrostatic precipitator, and/or
- prior to feeding the off-gas stream to a fan (103, 303a, 303b), and/or
- prior to feeding the off-gas stream to a cyclone (102, 302a, 302b).

4. The method according to any of the preceding claims, wherein residence time for the oxidation of the volatile organic compounds is at least 1 second.

5. The method according to any of the preceding claims, the method comprising determining the content of the volatile organic compounds in the off-gas stream on-line and adjusting the amount of the supplied ozone on the basis of the content of the volatile organic compounds.

6. A system (100, 300) for purifying an off-gas stream (101, 201, 301a, 301b) comprising particle matter and volatile organic compounds, the system (100, 300) comprising
- a dryer and/or a press used for wood-based board or panel production or a forming area and/or a curing oven of a mineral wool plant for producing the off-gas stream (101, 201, 301a, 301b) comprising particle matter and volatile organic compounds,
- a chemical dosing system (107, 307) arranged upstream of a wet electrostatic precipitator (104, 204, 304) for supplying ozone in a gas phase to the off-gas stream (101, 201, 301a, 301b), and
- the wet electrostatic precipitator (104, 204, 304) for removal of particle matter and volatile organic compounds from the off-gas stream (101, 201, 301a, 301b).

7. The system (100, 300) according to claim 6, wherein the chemical dosing system (107, 307) is arranged
- upstream of a quench (106, 206, 306a, 306b) of the wet electrostatic precipitator or a pre-scrubber preceding the wet electrostatic precipitator, and/or
- upstream of a fan (103, 303a, 303b), and/or
- upstream of a cyclone (102, 302a, 302b).

8. The system (100, 300) according to claim 6 or 7, wherein the chemical dosing system (107, 307) is arranged such that residence time for the oxidation of the volatile organic compounds is arranged to be at least 1 second.

9. The system (100, 300) according to any of the claims 6-8, further comprising at least one sensor configured to determine on-line the content of the volatile organic compounds in the off-gas stream, and a processor configured to control the chemical dosing system by taking into account the content of the volatile organic compounds in the off-gas stream determined by the at least one sensor.

10. Use of a system (100, 300) of any of the claims 6-9 for purifying an off-gas stream (101, 201, 301a, 301b) comprising particle matter and volatile organic compounds.

## Patentansprüche

1. Verfahren zum Reinigen eines Abgasstroms (101, 201, 301a, 301b), umfassend Partikel und flüchtige organische Verbindungen, wobei das Verfahren Folgendes umfasst
- Einleiten von Ozon in den Abgasstrom (101, 201, 301a, 301b), um die im Abgasstrom enthaltenen flüchtigen organischen Verbindungen zu oxidieren, und
- Unterziehen des Abgasstroms (101, 201, 301a, 301b) einem Nasselektrofilter,
wobei das Ozon in der Gasphase zugeführt wird,
das Ozon wird in den Abgasstrom (101, 201, 301a, 301b) eingeleitet, bevor der Abgasstrom (101, 201, 301a, 301b) einem Nasselektrofilter (106, 206, 306a, 306b) zugeführt wird, und
der Abgasstrom umfasst Abgase, die aus einem Trockner und/oder einer Presse stammen, die zum Herstellen von Holzwerkstoffplatten oder -paneelen verwendet werden, oder Abgase, die aus einem Formungsbereich und/oder einem Aushärtungsofen einer Mineralwolleanlage stammen.

2. Verfahren nach Anspruch 1, wobei das Ozon dem Abgasstrom (101, 201, 301a, 301b) vor einer etwaigen Wassereinspritzung oder Befeuchtung des Abgasstroms zugeführt wird.

3. Verfahren nach Anspruch 1 oder 2, wobei das Ozon dem Abgasstrom (101, 201, 301a, 301b) zugeführt wird
- vor dem Zuführen des Abgasstroms (101, 201, 301a, 301b) zu einem Quencher des Nasselektrofilters (106, 206, 306a, 306b) oder zu einem dem Nasselektrofilter vorgeschalteten Vorwäscher, und/oder
- vor dem Zuführen des Abgasstroms zu einem Ventilator (103, 303a, 303b) und/oder
- vor dem Zuführen des Abgasstroms zu einem Zyklon (102, 302a, 302b).

4. Verfahren nach einem der vorstehenden Ansprüche, wobei die Verweilzeit für die Oxidation der flüchtigen organischen Verbindungen mindestens 1 Sekunde beträgt.

5. Verfahren nach einem der vorstehenden Ansprüche, das Verfahren umfassend das Online-Bestimmen des Gehalts an flüchtigen organischen Verbindungen im Abgasstrom und das Anpassen des zugeführten Ozons auf der Grundlage des Gehalts an flüchtigen organischen Verbindungen.

6. System (100, 300) zum Reinigen eines Abgasstroms (101, 201, 301a, 301b), der Partikel und flüchtige organische Verbindungen enthält, das System (100, 300) umfassend:
- einen Trockner und/oder eine Presse, die zum Herstellen von Holzwerkstoffplatten oder -paneelen oder einem Formungsbereich und/oder einem Aushärtungsofen einer Mineralwolleanlage verwendet werden, um den Abgasstrom (101, 201, 301a, 301b) zu erzeugen, umfassend Partikel und flüchtige organische Verbindungen,
- ein chemisches Dosiersystem (107, 307), das einem Nasselektrofilter (104, 204, 304) vorgeschaltet ist, um dem Abgasstrom (101, 201, 301a, 301b) Ozon in der Gasphase zuzuführen, und
- den Nasselektrofilter (104, 204, 304) zum Entfernen von Partikeln und flüchtigen organischen Verbindungen aus dem Abgasstrom (101, 201, 301a, 301b).

7. System (100, 300) nach Anspruch 6, wobei das chemische Dosiersystem (107, 307) angeordnet ist
- stromaufwärts eines Quenchers (106, 206, 306a, 306b) des Nasselektrofilters oder eines dem Nasselektrofilter vorgeschalteten Vorwäschers, und/oder
- stromaufwärts eines Ventilators (103, 303a, 303b) und/oder
- stromaufwärts eines Zyklons (102, 302a, 302b).

8. System (100, 300) nach Anspruch 6 oder 7, wobei das chemische Dosiersystem (107, 307) so angeordnet ist, dass die Verweilzeit für die Oxidation der flüchtigen organischen Verbindungen mindestens 1 Sekunde beträgt.

9. System (100, 300) nach einem der Ansprüche 6 bis 8, ferner umfassend mindestens einen Sensor, der konfiguriert ist, um den Gehalt an flüchtigen organischen Verbindungen im Abgasstrom online zu ermitteln, sowie einen Prozessor, der konfiguriert ist, um das chemische Dosiersystem unter Berücksichtigung des durch den mindestens einen Sensor ermittelten Gehalts an flüchtigen organischen Verbindungen im Abgasstrom zu steuern.

10. Verwenden eines Systems (100, 300) nach einem der Ansprüche 6 bis 9 zum Reinigen eines Abgasstroms (101, 201, 301a, 301b), umfassend Partikel und flüchtige organische Verbindungen.

## Revendications

1. Procédé permettant de purifier un flux de gaz résiduaires (101, 201, 301a, 301b) comprenant de la matière particulaire et des composés organiques volatils, dans lequel le procédé comprend
- la fourniture d'ozone dans le flux de gaz résiduaires (101, 201, 301a, 301b) afin d'oxyder les composés organiques volatils compris dans le flux de gaz résiduaires, et
- la soumission du flux de gaz résiduaires (101, 201, 301a, 301b) à une précipitation électrostatique humide,
dans lequel l'ozone est fourni en phase gazeuse,
l'ozone est fourni dans le flux de gaz résiduaires (101, 201, 301a, 301b) avant l'acheminement du flux de gaz résiduaires (101, 201, 301a, 301b) vers un précipitateur électrostatique humide (106, 206, 306a, 306b), et
le flux de gaz résiduaires comprend des gaz résiduaires provenant d'un séchoir et/ou d'une presse utilisés pour la production de plateaux ou de panneaux à base de bois, ou des gaz résiduaires provenant d'une zone de formage et/ou d'un four de durcissement d'une usine de laine minérale.

2. Procédé selon la revendication 1, dans lequel l'ozone est fourni au flux de gaz résiduaires (101, 201, 301a, 301b) avant toute injection d'eau ou humidification du flux de gaz résiduaires.

3. Procédé selon la revendication 1 ou 2, dans lequel l'ozone est fourni au flux de gaz résiduaires (101, 201, 301a, 301b)
- avant l'acheminement du flux de gaz résiduaires (101, 201, 301a, 301b) vers un système de refroidissement du précipitateur électrostatique humide (106, 206, 306a, 306b) ou vers un pré-épurateur précédant le précipitateur électrostatique humide, et/ou
- avant l'acheminement du flux de gaz résiduaires vers un ventilateur (103, 303a, 303b), et/ou
- avant l'acheminement du flux de gaz résiduaires vers un cyclone (102, 302a, 302b).

4. Procédé selon l'une quelconque des revendications précédentes, dans lequel le temps de séjour pour l'oxydation des composés organiques volatils est d'au moins 1 seconde.

5. Procédé selon l'une quelconque des revendications précédentes, le procédé comprenant la détermination de la teneur en composés organiques volatils dans le flux de gaz résiduaires en ligne et l'ajustement de la quantité d'ozone fournie sur la base de la teneur en composés organiques volatils.

6. Système (100, 300) permettant de purifier un flux de gaz résiduaires (101, 201, 301a, 301b) comprenant de la matière particulaire et des composés organiques volatils, le système (100, 300) comprenant
- un séchoir et/ou une presse utilisés pour la production de plateaux ou de panneaux à base de bois, ou une zone de formage et/ou un four de durcissement d'une usine de laine minérale permettant de produire le flux de gaz résiduaires (101, 201, 301a, 301b) comprenant de la matière particulaire et des composés organiques volatils,
- un système de dosage de produits chimiques (107, 307) agencé en amont d'un précipitateur électrostatique humide (104, 204, 304) permettant de fournir de l'ozone en phase gazeuse au flux de gaz résiduaires (101, 201, 301a, 301b), et
- le précipitateur électrostatique humide (104, 204, 304) pour l'élimination de la matière particulaire et des composés organiques volatils du flux de gaz résiduaires (101, 201, 301a, 301b).

7. Système (100, 300) selon la revendication 6, dans lequel le système de dosage de produits chimiques (107, 307) est agencé
- en amont d'un système de refroidissement (106, 206, 306a, 306b) du précipitateur électrostatique humide ou d'un pré-épurateur précédant le précipitateur électrostatique humide, et/ou
- en amont d'un ventilateur (103, 303a, 303b), et/ou
- en amont d'un cyclone (102, 302a, 302b).

8. Système (100, 300) selon la revendication 6 ou 7, dans lequel le système de dosage de produits chimiques (107, 307) est agencé de telle sorte que le temps de séjour pour l'oxydation des composés organiques volatils est prévu pour durer au moins 1 seconde.

9. Système (100, 300) selon l'une quelconque des revendications 6 à 8, comprenant en outre au moins un capteur configuré pour déterminer en ligne la teneur en composés organiques volatils dans le flux de gaz résiduaires, et un processeur configuré pour contrôler le système de dosage de produits chimiques en tenant compte de la teneur en composés organiques volatils dans le flux de gaz résiduaires déterminée par l'au moins un capteur.

10. Utilisation d'un système (100, 300) selon l'une quelconque des revendications 6 à 9 permettant de purifier un flux de gaz résiduaires (101, 201, 301a, 301b) comprenant de la matière particulaire et des composés organiques volatils.
